# EUROPEAN PATENT APPLICATION

(11) **EP 4 207 016 A1**
(43) Date of publication of application: **05.07.2023**
(21) Application number: 22217355.1
(22) Date of filing: 30.12.2022
(51) Int. Cl.: G06Q 10/0631, G06Q 10/0639, G16H 10/20, G16H 50/20

(54) **PREDICTING EMPLOYEE WELLNESS USING PASSIVELY COLLECTED DATA**

(30) Priority: 30.12.2021 US 202163295498 P
(71) Applicant: Holmetrics Inc., Innisfail, AB T4G 0N9 (CA)
(72) Inventor: Ahmadi, Alireza, Calgary, T2N 0M7 (CA); Jetha, Nuran, Calgary, T3G 0A1 (CA); Norris, Devin, Calgary, T2E 1P7 (CA); Verity, Chad, Springbrook, T4S 0E5 (CA); Derkach, Lex, West Vancouver BC, V7S 2E3 (CA)
(74) Representative: Söllner, Udo

(57) **Abstract**

Systems and methods for predicting employee wellness using passively collected electronic data associated with one or more employees in a work environment using a model of the determined relationship between the collected survey data and predicted responses to a hypothetical survey directed to the one or more. Employee wellness of the one or more employees is predicted by applying predicted survey data to a framework for predicting employee wellness, the framework being based on actual survey data.

## Description

### TECHNICAL FIELD

The disclosed systems and methods relate to predicting employee wellness using passively collected data.

### BACKGROUND

Today, and into the foreseeable future, in the knowledge-based economy, employee performance is operational performance. With current technology, it has been challenging for leaders to marry employee experience and operational outcomes because the measurement tools for both are discontinuous and disjointed. Surveys and pro forma forecasts are fraught with assumptions and biases, relying heavily on point-in-time measurements and lagging indicators. Meanwhile the costs associated with disengagement, absenteeism, disability, and turnover quickly climb into the millions of dollars a year, while the lost revenue from the non-optimal performance is incalculable.

Measurement of psychosocial risk factors usually is done by utilizing surveys and employee interviews.

There are several problems associated with surveys alone, including low response rate, survey fatigue, socially desirable responses, and random answers. Also, conducting and analyzing surveys can take a considerable amount of time, making the survey outcomes outdated by the time the results are presented. At the same time, interviews are a great source of qualitative information, but they do not provide an opportunity to measure the progress of organizational change effectively. In addition, frequent surveying is resource-demanding and can lead to survey fatigue, which may result in reduced response rates.

### SUMMARY

There is provided in one embodiment a method of predicting employee wellness. Survey data is collected from one or more individuals. Electronic data associated with the one or more individuals is passively collected. A subset of extracted information is extracted from the electronic data to create extracted values. A relationship between the collected survey data and the extracted values is determined and a model of the relationship is stored in a database. Further electronic data associated with one or more employees is passively collected in a work environment. A subset of extracted information is extracted from the further electronic data to create further extracted values. Based on the model of the determined relationship between the collected survey data and the extracted values, responses to a hypothetical further survey directed to the one or more employees are predicted using the further extracted values to create predicted survey data. Employee wellness of the one or more employees is predicted by applying predicted survey data to a framework for predicting employee wellness based on actual survey data.

In various embodiments, there may be included any one or more of the following features of the method: the predicted employee wellness is displayed to a user on a dashboard; a change to a workplace environment is implemented based on the predicted employee wellness of the one or more employees; determining a relationship between the collected survey data and the extracted values further comprises using artificial intelligence and machine learning to predict employee responses to survey data; the further electronic data associated with the one or more employees further comprises one or more of: a number of employees in an organization, the relationship between the employee and other employees in the organization, internal communications between employees at the organization, external communication between employees and third parties, calendar event data, and time zone information; the further electronic data associated with the one or more employees comprises individual level data and organization level data; the further electronic data further comprises communication data; survey data is collected from the one or more employees, and employee wellness of the one or more employees is predicted based on collected survey data in addition to the predicted survey data; one or more recommended action items is provided based on predicted employee wellness of the one or more employees; the passive collection of further electronic data further comprises performing natural language processing on the content of the collected information; and the predicted survey data is verified in part by comparing the predicted survey data with the collected survey data.

There is provided in one embodiment a system for predicting employee wellness, the system comprises a processor configured to collect information from one or more devices associated with one or more employees in a work environment; a database operatively connected to the processor, the database configured to storing a model of a relationship between passively collected electronic data associated with one or more individuals and extracted values extracted from a subset of extracted information from the electronic data; the processor configured to derive the model of the relationship based on the collected survey data and the extracted values; the processor further configured to: passively collect further electronic data associated with one or more employees in a work environment from the one or more devices; extract a subset of extracted information from the further electronic data to create further extracted values; based on the model of the determined relationship between the collected survey data and the extracted values, predict responses to a hypothetical further survey directed to the one or more employees using the further extracted values to create predicted survey data; store the predicted survey data in the database; and predict employee wellness of the one or more employees by applying predicted survey data to a framework for predicting employee wellness based on actual survey data.

In various embodiments, there may be included any one or more of the following features of the system: a display operative connected to the processor and configured to displaying the predicted employee wellness on a dashboard; the processor determining a relationship between the collected survey data and the extracted values further comprises using artificial intelligence and machine learning to predict employee responses to survey data; the further electronic data associated with the one or more employees further comprises one or more of: a number of employees in an organization, the relationship between the employee and other employees in the organization, internal communications between employees at the organization, external communication between employees and third parties, calendar event data, and time zone information; the further electronic data associated with the one or more employees comprises individual level data and organization level data; the further electronic data further comprises communication data; the processor is further configured to: collect survey data from the one or more employees, and predict employee wellness of the one or more employees based on collected survey data in addition to the predicted survey data; the passive collection of further electronic data further comprises performing natural language processing on the content of the collected information; and the processor is configured to verify the predicted survey data in part by comparing the predicted survey data with the collected survey data.

These and other aspects of the system and method are set out in the claims.

### BRIEF DESCRIPTION OF THE FIGURES

Embodiments will now be described with reference to the figures, in which like reference characters denote like elements, by way of example, and in which:
Figure 1 is a flow chart showing collected data that is processed and modelled based on a survey framework.
Figure 2 is a flow chart showing an embodiment of a method of predicting employee wellness.
Figure 3A, 3B and 3C is a flow chart of information stored in a centralized database.
Figure 4A and 4B is a schematic diagram showing an end-to-end process of an embodiment of the methods disclosed herein.
Figure 5 is an exemplary embodiment of an intranet widget for use with embodiments of the methods disclosed herein.
Figure 6 is an exemplary embodiment of a Microsoft Teams^{™} adaptive card for use with embodiments of the methods disclosed herein.
Figure 7 is an exemplary login page.
Figure 8 is an overview section of a dashboard.
Figure 9 is a display of the dashboard showing a list of growth factors.
Figure 10 is a display of the dashboard showing an assessment of risk factors.
Figure 11 is a display of the dashboard showing results of the survey questions.
Figure 12 is a display of the dashboard showing change of the metrics over time.
Figure 13 is a display of changes in trends associated with the workplace wellness of an employee.
Figure 14 is an exemplary system which can implement embodiments of the methods disclosed herein.
Figure 15 is a display of the dashboard showing changes of the metrics over time.

### DETAILED DESCRIPTION

Embodiments of the methods and systems disclosed herein may utilize a wide range of machine learning and natural language processing algorithms to predict employees' scores according to the Copenhagen Psychosocial Questionnaire (COPSOQ) inventory. The method may use a survey only once at the beginning for validation to ensure that the predictions have an acceptable accuracy level. Apart from removing the need for any follow-up survey or interview, the approach provides real-time results and updates. It shows the trend over time and helps with further insights and comparisons.

The main steps in embodiments of the methods disclosed herein start with collecting data. Then the valuable information will be extracted from collected data to be passed for prediction. Predictive models use this information to predict the psychological risk factors. After processing, the results are visualized into a dashboard.

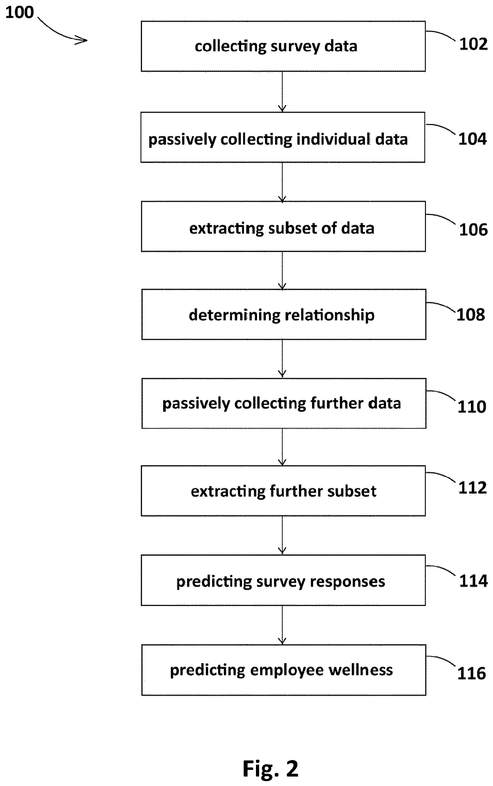

As shown in Fig. 2, there is a method of predicting employee wellness 100. The method comprises collecting survey data from one or more individuals at 102. Electronic data associated with the one or more individuals is passively collected at 104. A subset of extracted information from the electronic data is extracted at 106 to create extracted values. A relationship is determined at 108 between the collected survey data and the extracted values and model of the relationship is stored in a database.

Further electronic data associated with one or more employees in a work environment is passively collected at 110. The passive collection of further electronic data may be conducted by performing natural language processing based on the content of the collected information.

A subset of extracted information from the further electronic data is extracted at 112 to create further extracted values. The further electronic data associated with the one or more employees may be one or more of: a number of employees in an organization, the relationship between the employee and other employees in the organization, internal communications between employees at the organization, external communication between employees and third parties, calendar event data, and time zone information. The further electronic data associated with the one or more employees may be one or both of individual level data and organization level data. The further electronic data may be communication data.

Based on the model of the determined relationship between the collected survey data and the extracted values, responses to a hypothetical further survey directed to the one or more employees is predicted at 114 using the further extracted values to create predicted survey data. Employee wellness of the one or more employees is predicted at 116 by applying predicted survey data to a framework for predicting employee wellness based on actual survey data. The predicted employee wellness may be displayed to a user, such as a customer, on a dashboard. The predicted survey data may be verified in part by comparing the predicted survey data with the collected survey data.

A change may be implemented to a workplace environment based on the predicted employee wellness of the one or more employees. Changes could include additional time off, providing access to mental health services, changes in job title or responsibility, changes in management or any other business tools which have an impact on employee wellbeing. The step of determining a relationship between the collected survey data and the extracted values may include using one or both of artificial intelligence and machine learning to predict employee responses to survey data.

The method may also include the additional steps of collecting survey data from the one or more employees, and predicting employee wellness of the one or more employees based on collected survey data in addition to the predicted survey data.

The method may provide data for an organizational consultant to provide recommendations and build action items. The method may provide one or more recommended action items based on predicted employee wellness of the one or more employees. The recommended action items may be provided to the management of a workplace environment, which may result in one or more changes being implemented to the workplace environment. Recommendations may be reviewed by a mental health professional.

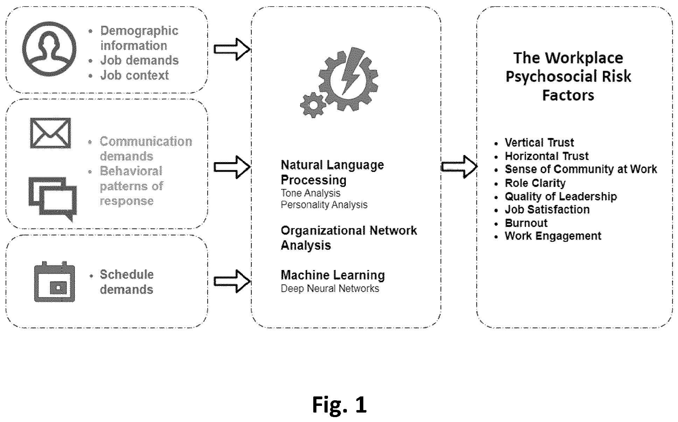

A high-level overview of certain steps taken by an embodiment of the methods described herein is shown in Fig. 1. Various information is collected from various sources relating to one or more individuals at an organization. The information may include demographic information, job demands and job context. Communication to and from the individual may be collected, including viewing details of communication demands and behavioural patterns of response. Schedule demands for the individual may also be tracked. All of this information may be tracked for multiple individuals across the organization. The collected information may be analyzed by various processes, including Natural Language Processing, including sentiment analysis and toxicity classification, Organizational Network Analysis and Machine Learning, including deep neural networks, to map to work environment assessment survey data such as provided in a COPSOQ survey. The data collected and the survey information that is collected or predicted may be varied depending on the type of information that is desired to be tracked and correlated in each particular work environment.

Additional details of embodiments of the methods disclosed herein are set out in the description below.
1- Data Collection: The communication and scheduling data available on the set of software tools used by an organization is accessed. In many cases, the richest and vital information is embedded in messages that employees send over e-mails or chats and meetings they set in their calendars. However, there are sensitive data parts in these sources which cannot be stored in any other place. In these cases, the method performs the corresponding information extraction process (from item 2 below) in real-time and stores the processed data. The exemplary collected data that is discussed in certain embodiments herein have been found to provide useful correlations with metrics obtained from COPSOQ surveys. However, any types of data that are available on a device to be extracted could hypothetically used by embodiments of the system and method disclosed herein.
2 - Accessing Raw Data and Preparing Them: The accessed data will be examined using different approaches to provide valuable information that can contribute to the accurate prediction of psychological risk factors. Examples of approaches used by embodiments of the methods are explained below.
   2.1 - Direct Extraction: Initially, useful information will be extracted directly. For communication data, the frequency of messages between different people inside/outside the organization over different periods of the day/week is extracted. It also includes the reactions (emojis) used in communications. Scheduling data, such as the length of meetings, number of participants, starting and ending time, will be extracted.
   2.2 - Semantic Analysis: Textual communications are semantically analyzed to provide valuable information. Initially, to utilize this data, in some embodiments, Natural Language Processing tools may be used, such as: IBM Watson^{™} Tone Analyzer or Amazon^{™} Comprehend, which analyze the tone of messages with four scores, such as Positive, Negative, Mixed, and Neutral. In other embodiments, other scores for emotions could be detected, including: Anger, Fear, Joy, Sadness, Analytical, Confident, Tentative. Other attributes of the messages are extracted such as words count, characters count, question marks count. Words count will utilize custom dictionaries that include words that correlate with psychological constructs related to psychosocial risk factors. The frequency of words occurrence in these messages will be taken into account. Toxicity analyzers may also be used to assess the level and frequency of toxic communication.
   2.3 - Organizational Network Analysis: By considering the chat and e-mail messages sent between the individuals, an Organizational Network Analysis (ONA) may be conducted. ONA is an approach that helps to investigate the organization's social structures and each individual's position in those structures. It provides information about how each employee is connected to all others, for example, including determining if he/she is included or excluded from teams.
   2.4 - Demographic Information: embodiments of the methods and systems may incorporate the individuals' demographic information and make it anonymous. A part of this information comes from the organization software tools and another part from the initial survey. Embodiments of the methods and systems may extract the context and activities for each individual's job using an extensive database of job descriptions developed by The Occupational Information Network (O*NET). This type of information allows the system to consider job responsibilities and a difference in behavioral patterns caused by them. Therefore, it helps with the generalizability of the models.
3 - Prediction Process: The preprocessed data will be fed into the trained machine learning models. These models are trained using a variety of machine learning algorithms. The initial survey results are considered as the labels of the preprocessed data to build the training set. The models start getting trained on the information from the last step in order to learn patterns and relationships between features and labels. The models then make the best possible mapping from passively collected work-related data to the real-world survey answers.
   The prediction process is accomplished by determining a correlation between communication data and survey data. Customized Python scripts may be used to determine those correlations by utilizing machine learning libraries. The machine learning algorithms determine correlations between the communication data and the surveys using customized algorithms derived from known machine learning algorithms. Labels of data to input into the machine learning algorithms are derived from onboarding surveys. In the initial prediction process, various input may be entered into the machine learning algorithms to determine which input data is important. Various types of survey questions and various types of communication data may be entered and analyzed by the algorithms to determine which survey answers correlate to which communication data. The algorithms may be continuously updated throughout the use of the method and system, not just during the initial setup process. Various machine learning algorithms may be used. For example, Pandas may be used as the data processing library. The libraries and functions used to determine these correlations may be derived from known systems. Correlations from the survey data may be determined by extracting known COPSOQ survey questions and correlations known in the art. Factors that are analyzed may be derived from existing COPSOQ manuals. The reliable and reasonable mapping in the models equips the system with the capability of continuous assessment of survey questions, and subsequently the workplace psychosocial risk factors.
4 - Visualization: The predicted psychological factors are visualized to provide interpretable actionable results. The outcomes are plotted over time to demonstrate the trend for further comparison.

The collected raw data will go through the preprocessing steps. The resulting extracted information contributes to the prediction. The prediction would be performed using a portfolio of models at different levels. The algorithms determine equations that provide weighted correlations between the input and output of the collected data and the answers. The equations determined through these models allow the method and system to predict how an individual would answer the survey questions. For each set of questions, different algorithms may be used. The determined equations may have simple linear weight correlations or may use more complicated functions. Particular algorithms may be used to get a baseline and if an algorithm appears to provide useful data, then it may be selected to be used in the process. Algorithms that do not provide good predictions may be discarded. The process to generate the models may be done iteratively and may be continuously reviewed and updated. Eventually, the mean of resulted scores is listed as the outcomes and prepared for the visualization.

Some embodiments of the systems and method disclosed herein may have one or more of the following features:
a. Cost-effectiveness: the approach eliminates the need for surveying and saves hours of employees' time for their daily work.
b. Time-effectiveness: Following this approach, the result will be accessible up-to-date, and the delay in the survey report will be addressed.
c. Continuity: The situation is under continuous monitoring with monthly well-being reports, while surveying (and other similar approaches) are not optimized enough to be performed monthly.
d. Generalizability: The results encompass the whole company and not only people who are taking surveys. This overcomes typical survey problems such as low response rate, survey bias, socially desirable answers, survey fatigue, etc.

The following data may also be used in embodiments of the methods and systems disclosed herein:
a. Data from HR software tools.
b. Data from project management software tools.
c. Data from environmental sensors such as light intensity, noise, temperature, and humidity.
d. Data from wearable devices such as activity level and heart rate.

Embodiments of the systems and method disclosed herein may provide real-time employee experience metrics daily via cloud-based dashboards to leaders and managers of companies that can be highly customized and filtered. The dashboards offer diagnostic tools, and leading indicators.

Embodiments of the method allows companies to upload key performance indicators using pre-built software integrations and comma-separated values (CSV) which are analyzed by the software to discover trends, patterns, and correlations between the organization's metrics and the organization's key performance indicators (KPIs).

Data may be used to attempt to predict organizational KPIs based on metrics that are improved in real-time or near real-time through the utilization of embodiments of the disclosed platform.

Data may be stored safely by implementing the following:
a. The methods do not collect or store any sensitive data from the customers.
b. The methods do not access any personal, private data from the customer's employees.
c. The only data the methods assess is data owned by the company.

Examples of data privacy and security practices that may be used include:
a. Data Aggregation
b. Data Autonomy
c. Data Sovereignty
d. The analysis is only accessible by the customer
e. Segregated storage
f. Regular security reviews

Instead of using surveys, embodiments of the methods and systems connect to the tools that teams use every day, such as Microsoft 365^{™}, Google Workspace^{™} and Slack^{™} and analyze thousands of data points in real-time. The AI maps those data points to a globally recognized framework for assessing workplace wellbeing, the Copenhagen Psychosocial Questionnaire (COPSOQ). Embodiments of the system and method may use the evaluations made by the software to accurately assess an entire organization across eight metrics: Vertical Trust, Horizontal Trust, Sense of Community at Work, Role Clarity, Quality of Leadership, Job Satisfaction, Burnout, Work Engagement. These are a subset of certain factors derived from COPSOQ surveys. These factors have been shown to correlate with obtained communication data discussed herein. Other metrics may be used if different subsets of data are obtained from the workers. Based on analysis conducted to date, it was determined that the eight factors provided above provide the most useful correlations to the communication data currently obtained at the work site. The other COPSOQ factors do not provide information with currently obtained data that matches the level of usefulness. These additional COPSOQ factors may become more useful if additional or different metadata is obtained, for examples based on institutional data in addition to communication data or from a different subset of communication data. Different correlations and algorithms and metrics may be developed based on the metadata obtained through the software.

Embodiments of the system and method may provide real-time employee experience metrics on a daily basis via cloud-based dashboards to leaders and managers that can be highly customized and filtered based on numerous demographics including department, location, tenure, remote/hybrid/office work type, age, gender, race and ethnicity among others. Embodiments of the system and method are called Hölmetrics^{™} in this document. The dashboards may provide diagnostic tools, leading indicators and recommendations vetted by certified occupational therapists to improve employee experience.

Embodiments of the system and method may allow companies to upload key performance indicators using pre-built software integrations and CSV that are then analyzed by the software to discover trends, patterns and correlations between the organization's Hölmetrics^{™} and those KPIs.

There are basic human psychological needs inherent in any organization that, if met, prevent critical incidents, promote general wellbeing, and increase productivity in any work environment (Canadian Standards Association, 2013). Failure to acknowledge these needs at a basic level prevents individuals from engaging in higher-level needs and can lead to distress that negatively affects individual, financial, and organizational goals (Canadian Standards Association, 2013). Hölmetrics^{™} empowers leaders to address these needs. Example metrics that may be indicators of human team performance within the influence of any management and leadership team including the following: Vertical Trust, Horizontal Trust, Sense of Community at Work, Role Clarity, Quality of Leadership, Job Satisfaction, Burnout, Work Engagement.

### Burnout

Burnout concerns the degree of physical and mental fatigue/ exhaustion of the employee.

### Work Engagement

The most frequently used words to describe employee engagement are involvement, commitment, discretionary effort, collaboration, motivation, and performance (Bridger, 2015). Engagement is evidenced by the experience of enjoyment, connectedness, inspiration and personal commitment of an employee in their approach to work (Canadian Standards Association, 2013). Furthermore, engagement is a consequence of organizational practices that intentionally protect and promote employees' psychological wellbeing (Canadian Standards Association, 2013).

Engagement is a thoroughly researched dimension of the mental health of an organization and has a considerable impact on the individual. Engagement acts as a buttress to emotional wellbeing for individuals dealing with other significant life stressors (Gallup, 2013). Alternatively, lack of employee engagement is correlated with worry, depression and uneasiness, absenteeism, reduced productivity, and burnout (CIPD, 2010; Bridger, 2015). Efforts to encourage engagement in an organization can lead to enjoyment, improved quality of life and decreased incidence of chronic illness (Gallup, 2013).

### Job Satisfaction

Job Satisfaction deals with the employees' experience of satisfaction with various aspects of work.

Role Clarity deals with the employee's understanding of her or his role at work, i.e., the content of the tasks, expectations to be met, and her/his responsibilities.

Quality of Leadership deals with the following higher managers' leadership in different contexts and domains.

Community at Work concerns whether there is a feeling of being part of the group of employees at the workplace, e.g., if employees relations are good and if they work well together.

Vertical Trust deals with whether the employees can trust the management and vice versa. Vertical trust can be observed in the communication between the management and the employees.

Horizontal trust deals with whether the employees can trust each other in daily work or not. Trust can be observed in communication in the workplace, e.g. if one can freely express attitudes and feelings without fear of negative reactions.

### Overview Dashboard

The Overview Dashboard may provide an overview of the Hölmetrics^{™} at an organizational level. A Hölmetrics^{™} Score may be presented with an aggregated score capturing the organization's whole health.

Gauges present an assessment of Vertical Trust, Horizontal Trust, Sense of Community at Work, Role Clarity, Quality of Leadership, Job Satisfaction, Burnout, Work Engagement. The Hölmetrics^{™} factor scores may be based out of 100. Each Hölmetrics^{™} presents an up-to-date score along with the historical trend. The scores may be presented in various formats so long as they are useful for the viewer. A score out of 100 is only one way in which the information may be presented. So long as useful information can be obtained from the displayed results, various different formats of presentation are possible.

### Management Consulting Dashboard

The management consulting dashboard presents a powerful tool that empowers management consultants to assess high level metrics across multiple companies that the management consultant is working with. Various different subsets of information can be displayed in different dashboards, depending on the target audience. Information should only be presented so long as the person receiving the information does not obtain confidential information about an individual or organization that they should not have access to. The various dashboards can assess with pinpoint accuracy where inside an organization they can have the most impact and measure that impact over time, down to the department level.

Inside the Management Dashboard, leaders can create custom datasets using prebuilt filters, enabling them to understand the experience of employees.

The Management Dashboard can be custom-tailored to the users, presenting only information relevant to the individual user's context.

### Leading Indicators

Along with a detailed breakdown of the assessment based on customizable datasets, embodiments of the system and method also provide the leading indicators of that Holmetric, providing the basis for the assessment. Leading indicators will help the employer understand why the assessment is what it is. Leading Indicators may be presented in descending order.

### Recommendations

A person such as an organizational development consultants, organizational psychologist, or an HR professional may be able to use any information determined and presented in the Hölmetrics^{™} system to provide recommendations. The data allows for informed decisions to be provided for use in the workplace.

### Integrations

Embodiments of the system and method have integrations for Microsoft365^{™}, Google Workspace^{™}, and Slack^{™}, with pre-built integrations into most human resource and human capital management systems, including WorkDay^{™}, SAP Success Factors, BambooHR^{™}, project management software and many more readily available through third-party service providers.

### Modelling

Hölmetrics^{™} tracks approximately 75 different metrics in real-time on every employee inside the company. Examples of these metrics are set out later in this disclosure. These metrics may be pulled nightly using the data pipeline. Although the data may be pulled nightly, it may also be done at any time. Pulling data once a day ensures that up-to-date information is obtained without the need to continuously monitor the input data. Nightly reviews provide a good amount of time to review. Different timings and frequency of pulling data may be used depending on the needs of the customer. The data may be obtained using customized modifications of pre-existing widgets available through Microsoft^{™} and GSuite^{™} APIs. The data is anonymized, aggregated, and kept confidential. Embodiments of the methods and systems only assess data that is owned by the company, that is, the customer. Embodiments of the system and method do not access any personal, private data of any kind. Hölmetrics^{™} accesses the existing data for analysis to provide the customer with real-time insights, meaning the systems and methods do not store or collect any sensitive data of any kind. Instead embodiments of the system and method assess the data on the customer's server without storing personally identifiable information on the system's server. For example, the method may access customer data via Microsoft^{™} and GSuite^{™} APIs, and after removing personally identifiable information and censoring message content, that information is stored in one or more databases.

### Introduction

Hölmetrics^{™} aims to improve employee wellbeing and operational performance by providing organizations with real time insights on how employees are doing across a number of different workplace psychosocial risk factors. Hölmetrics^{™} ties into the data from the tools that employees use every day to understand the wellbeing of an organization in real time, eliminating the need for employee engagement surveys. By providing insights in real time, organizations are better positioned to understand the relationships between employee well being and operational performance and establish methods for improving conditions ahead of time thereby minimizing operational impact and improving overall performance.

### Components of Holmetrics^{™}:

### Model Validation Tools

The applicant has conducted an extensive review of risk factor measurement tools to use as the framework behind software and data science modelling. In a preferred embodiment, the system uses the third version of the Copenhagen Psychosocial Questionnaire (COPSOQ), which is an assessment tool developed by the international network of researchers who study workplace risk factors and employee wellbeing. This assessment tool was selected due to its alignment with the National Standard for Psychological Health and Safety in the Workplace and good psychometric properties. The COPSOQ had been applied in more than 400 peer-reviewed articles (Burr et al., 2019), and it is available in more than 25 languages. The COPSOQ factor structure was recently verified among Canadian workers (Ramkissoon et al., 2019). In other embodiments other questionnaires may be used.

As the final goal is to predict the risk factors listed by COPSOQ, the system needed a way to collect submissions from employees to prepare the data for and validate the machine learning modelling. For this reason, the systems and method use two methods in leveraging COPSOQ.
a. Development of a pulse survey widget that can be installed on any website (typically the customers intranet) which will record pulse survey questions in a condensed form of COPSOQ and send the employee responses back to the systems through API calls.
b. Development of an electronic version of the COPSOQ questionnaire which can be delivered to employees on a one-time basis. Examples of the types of electronic versions are discussed in more detail below.
c. Development of a Pulse survey system which will send daily questions to users through an app published in the Microsoft Teams^{™} store.

Typically, customers will decide on one of these tools during the Hölmetrics^{™} onboarding process.

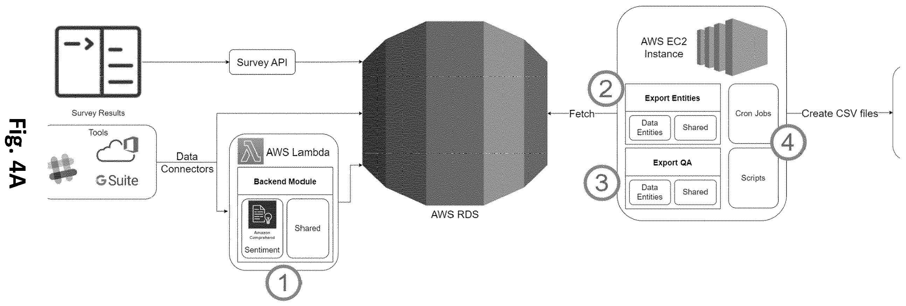

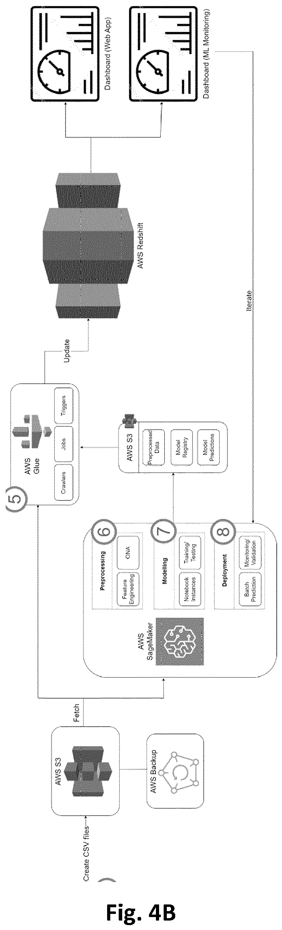

Figs. 4A and 4B show a diagram which outlines the end to end process. Figs. 4A and 4B represent a single diagram that is split across two pages. This process is explained in more detail below.

### Feature Extraction

The extracted features, organized as the data table, are used in two streams. First, they will be fed into the developed model. Secondly, they will be converted to report data to be used as a dashboard in the Hölmetrics^{™} Web Application.

### Modelling

For modelling, a series of Machine Learning algorithms may be used. Through an iterative process, the features are modified and their importance are assessed. Then the model(s) are trained, validated, and eventually the feedback is used for further modification in feature engineering and model design. The final model will make the best possible mapping from passively collected work-related data to the psychological risk factors. The reliable prediction of the model equips the system with continuous assessment of the workplace psychosocial risk factors.

### Hölmetrics^{™} Web Application

The Hölmetrics^{™} Web App serves as the customer facing front end of the entire solution. From this front end users/employees of the customers can view relevant data including scoring on the Hölmetrics^{™} workplace psychosocial risk factors, trending, and drill down and filter capability. The web app also provides a resource centre for relevant documentation and videos as well as methods to reach out for application support.

### Data Connectors and Unified Data Model

Embodiments of the system and method use purpose built data connectors for Microsoft^{™} Graph, G-Suite^{™}, and Slack^{™} APIs which connect and ingest data from these data sources while extracting relevant data points and processing the employee data on the fly. In Fig. 4A an exemplary embodiment with G-Suite^{™} is shown. The data from these data sources includes relevant chat, email and calendar event data which is processed and stored in a unified data model in the operational databases. The unified data model shown in Fig. 4A allows data from all the customers (regardless of data source) to be stored in a central location, to be accessed by the data modelling routines and data pipelines.

### Data Pipelines and Data Warehouse

Embodiments of the system and method use data pipelines to extract, transform and load processed customer data into the data warehouse. Data pipelines have also been developed to extract model result data and automatically load into the data warehouse. All data pipelines may be scheduled to run on a nightly basis (or manually). The data warehouse is used to provide clean reportable data that is used by the Hölmetrics^{™} Web App and dashboards.

### Introduction

Embodiments of the system and method may improve employee wellbeing and operational performance by providing organizations with real time insights on how employees are doing across several different psychosocial risk factors. Unlike a survey-based approach, Hölmetrics^{™} relies on passive data collection and extraction, as well as artificial intelligence (AI) and machine learning (ML) to predict how employees would respond to questions on surveys related to their mental health and the health of their workplace.

By providing Hölmetrics^{™}, the software provides metrics which would be the equivalent of surveying all employees within an organization every single day, with data refreshed, aggregated, and presented on the dashboards each night. This can be an extremely powerful tool to see the day-to-day changes in the workplace, understand the impact of organizational changes at a deeper level and understand the relationships between employee well-being and operational performance. In addition to this, by using a Hölmetrics^{™} method, embodiments of the system and method can reduce the costs of conducting surveys, improve response rates (passive data would be at or near 100%), and greatly improve the timeliness of the results (it can take months to compile and make sense of the results from a traditional survey).

### Components of Hölmetrics^{™}:

### Data Connectors

Hölmetrics^{™} uses a method of passive data collection to extract key data points from communication software. The data connectors are purpose built to tap into tools such as Office 365^{™}, Google^{™} Workspace (G-Suite^{™}), and Slack^{™}. The connectors are written in Node.JS^{™} and are pulling data incrementally for each customer, in an asynchronous fashion to maximize speed and efficiency of the data ingestion and processing. The connectors are hosted on an Amazon^{™} EC2 instance and are scheduled to run nightly for all customers.

Embodiments of the system and method use endpoints provided by these communication tools to gather data related to:
- Employees in an organization
- Email communication
- Individual and Group chat communication
- Team and Channel chat communication
- Calendar Event data
- Employee time zones
- Managers of employees
- Employee groups

The connectors are intended to run nightly however can be run at any time to incrementally process and load new data from the customers. Embodiments of the system and method have also purpose-built filter and censor routines, which remove duplicate (or unnecessary) data and censor or obfuscate private/sensitive information. Raw email, chat, and calendar event content is never stored on any of the systems.

The following is a list of censored data:

### Calendar Events:

- Attendee email addresses
- Organizer email addresses
- Meeting subject
- Meeting body and body preview
- Meeting URL
- Conference call data
- Meeting location
- Attachment information

### Email Communication:

- From/To/CC/BCC/ReplyTo Email addresses
- Message body
- Filenames/attachment information
- Subject

### Chat Communication:

- User information
- Device information
- Application information
- Message body
- Message attachment information
- Urls/Links
- Subject
- Chat summary

### In-Memory Processing

A product requirement in many cases is to ensure that no sensitive data is stored on the servers. Embodiment of the system and method use custom built processing routines for O365^{™}, G-Suite^{™}, and Slack^{™} that extract key features from the email, chat, and calendar data that are required for the AI/ML routines. These routines are written in Python and are hosted in serverless Amazon^{™} Web Services (AWS) Lambda functions. The data connectors call the API endpoint for the Lambda functions and pass the communication data (email, chat, calendar). This processing step utilizes AWS Comprehend to perform Natural Language Processing (NLP) on the content of communication data, allowing the system to extract useful information without storing anything private. The Lambda function then returns the resulting feature set and values in a single JSON object which is stored on AWS Relational Database Service along with the censored source communication data.

### Unified Data Model

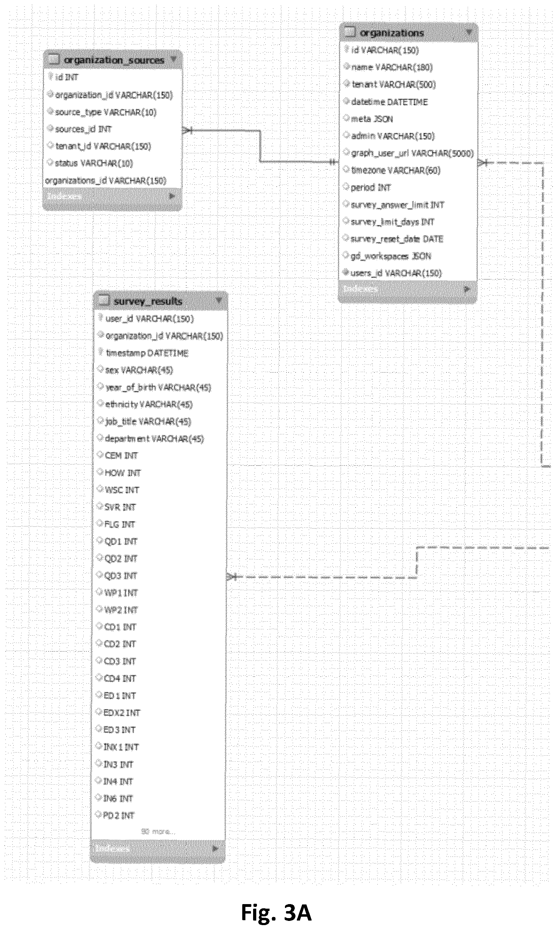

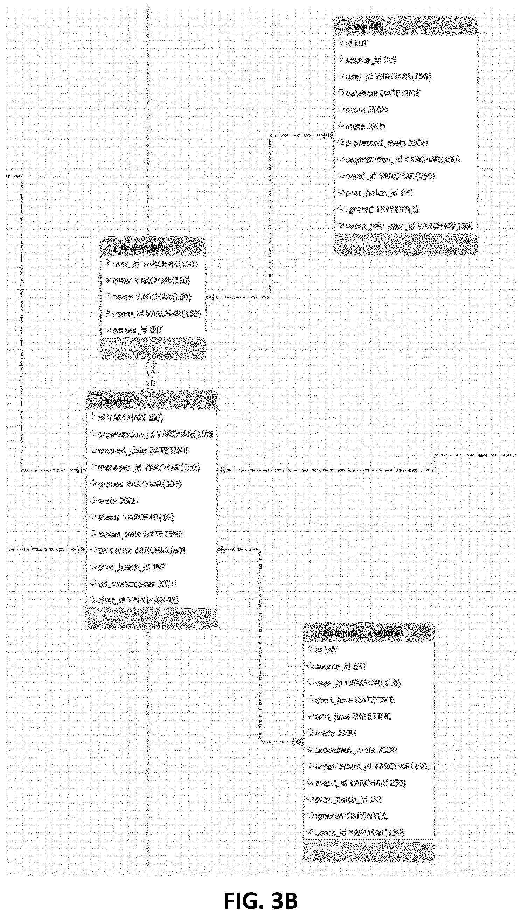

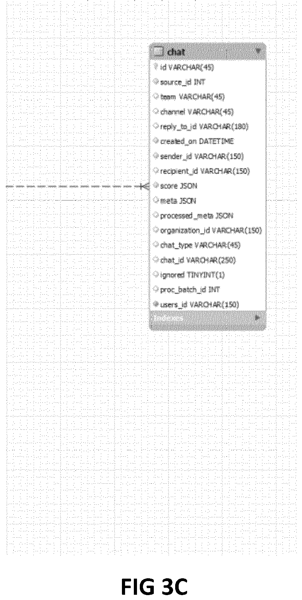

All data collected by the connectors and by Hölmetrics^{™} Pulse (described further below) is processed and stored in a centralized database using the unified data model (see Figure 3). The unified data model allows for data from all sources (Office 365^{™}, Slack^{™}, Google^{™} Workspace) to be processed and stored in the same repository for downstream processing as inputs to the AI/ML models and the Hölmetrics^{™} front end web application/dashboards. Another Python module hosted on AWS EC2 is responsible for accessing the processed anonymized data from RDS (described above), cleaning & parsing it, and creating daily scheduled entity reports which are then saved in Amazon^{™} Simple Storage Service (AWS S3) (data lake) and Redshift (data warehouse).

In Figs. 3A, 3B and 3C there is set out a list of entities in the core data model and a short description of what they contain:

| **Entity Name** | **Description** |
|---|---|
| Users | Contains the id and necessary information on employees (users) |
| Users_Priv | Contains a mapping of user id to email address. This table can only be accessed by the database admins. |
| Emails | Contains processed email data from Office 365^{™} and Google^{™} G-Suite^{™} |
| Chats | Contains processed chat data from Microsoft Teams^{™} and Slack^{™} |
| Calendar_Events | Contains processed event data from Microsoft^{™} Outlook 365^{™} and Google Workspace^{™} |
| Organizations | Contains organizations (customers) |
| Organization_Sources | Contains all data sources applicable to each organization |
| Survey Results | Contains employee submitted survey results from Hölmetrics^{™} Pulse |

### Feature Engineering

As mentioned above, embodiments of the system and method collect data about the emails, chats, and calendar events of an organization through their existing communication software and store it in separate daily reports, which are called entities. Using another Python module (through AWS SageMaker^{™}) that processes various combinations of the data entities reports, embodiments of the system and method engineer many different features for the individuals of the organization. These features are numerical attributes which represent the communication and event patterns for members of an organization. They are extracted at the daily level, stored in tabular format on AWS S3, and then aggregated to a certain time frame to be used for dashboards and modelling. By selecting features that are informative and relevant to the task at hand, they will be useful as input for the machine learning models.

Below is a detailed list of features the module currently engineers. Note that 'internal' refers to communication within a specific organization, and 'external' refers to communication with individuals outside the user's organization.

### Calendar Events:

- Number/length of events organized
- Number/length of external events
- Number/length of internal events
- Number/length of 1-on-1 events

### Email Communication:

- Number of outgoing emails (external, internal, and total)
- Number of incoming emails (external, internal, and total)
- Number of emails outside regular working hours
- Number of 1-on-1 emails
- Number of characters in subject/body
- Number of words in subject/body
- Number of question marks in subject/body
- Sentiment scores of email body (content is scored on a scale between 0 and 1 for four different sentiments: positive, negative, neutral, and mixed, using AWS Comprehend)

### Chat Communication:

- Number of messages sent
- Number of messages received
- Number of 1-on-1 messages
- Number of messages outside regular working hours
- Number of reactions to the user's messages (e.g., number of `like' reactions)
- Number of times the user is mentioned
- Number of times the user is replied to
- Number of characters in messages
- Number of words in messages
- Number of question marks in messages
- Sentiment scores of messages

These features may be initially determined by trial and error by conducting a cross-correlation between the features and the results of survey data. The features provided above are examples of data which may be useful. Other features could also be considered. Any data that can be obtained from the individuals which can be translated into raw data is information that could be useful for the system. Any correlations with survey data that can be extracted may be useful to track and to analyze. Additional data could be extracted. The data that is extracted and the survey questions that are reviewed may be determined iteratively and can be varied. Additional features could be considered including toxicity which could be determined and evaluated from the data collected. As machine learning and natural language tools advance, additional information may be extractable from the data that is obtained and used within the systems and methods described herein.

### Organizational Network Analysis:

To extract even more information embedded in messaging patterns, embodiments of the system and method conduct Organizational Network Analysis (ONA). ONA is an approach that helps to investigate the social structure of an organization and its communication patterns. It provides the system information about how each employee is connected to everyone else - are they a central part of how the organization communicates, or are they ostracized? These and other questions are answered through ONA by constructing a network based on the emails and chats that flow through each organization. Then embodiments of the methods and systems can extract a set of mathematically defined centrality measures which embodiments of the methods and systems use as features:
- Degree centrality
- In-degree centrality
- Out-degree centrality
- Closeness centrality
- Betweenness centrality

These centrality measurements are well-defined in graph theory. The network may be predicted by looking at the communication model to build a model graph of the network. The ONA may be conducted for each organization separately. The network is determined based on features of the communication data. The ONA may be generated automatically by the method to create estimates of centrality based on the data. The ONA analysis may be run and updated once a week. Changes may be made over time based on the way the organization is communicating. In determining the ONA of the organization, the models may look at chats and emails to determine the centrality indicators identified through the conversations. Centrality is determined for each organization. The resulting models of centrality are used as a feature to predict employee wellness. The calculations of connectivity may not be presented directly to the user.

All the above features, organized in data tables and stored on S3, are used in multiple streams. They are either used as input for training the machine learning models or fed into a developed machine learning model to be predicted on. Simultaneously, they will be converted to report data to be used for generating the dashboard in the Hölmetrics^{™} Web Application.

### Modelling

To reiterate, the overall goal of Hölmetrics's^{™} application of machine learning is to be able to confidently predict how an individual working at a certain organization would answer a psychological survey question based solely on their communication patterns obtained through passive data collection. The nature of this problem is thus one of `supervised learning' - where models utilize existing data to learn a function that can take input features (communication features) and map them to a desired output (survey answers), referred to as 'labels'.

Then, in order to train the models, embodiments of the system and method may first require real feature and label data - communication features for a specific employee combined with their actual responses to the COPSOQ survey questions. Real survey data has been gathered through Hölmetrics^{™} Pulse (described below). An individual answer to a survey question given through Pulse is stored in S3, and subsequently mapped to its corresponding communication features using unique organization and user identifiers.

In some embodiments, there are a total of 27 different questions that the ML models predict answers to. These questions may fall into one of 8 categories (or factors) derived from COPSOQ factors as discussed above. Each question in the questionnaire may have a possibility for 1 of 5 answers (typically some variation of the format always, often, sometimes, seldom, never). Thus, in some embodiments there are 27 machine learning models (one for each question), each which will predict 1 of the 5 possible answers for its specific question. The models may be derived during the training process and these same models may also be used to predict answers. The training process which determines correlations between actual survey answers and obtained data may then be applied after the correlation equations are determined to create predicted answers to the survey questions based on the obtained data. Different selections and total numbers of questions can be used in other embodiments.

Questions that may be used include the following:
a. How often have you been emotionally exhausted?
b. How often have you felt worn out?
c. How often have you been physically exhausted?
d. How often have you felt tired?
e. Do you know exactly what is expected of you at work?
f. Does your work have clear objectives?
g. Do you know exactly which areas are your responsibility?
h. Regarding your work in general. How pleased are you with your work prospects?
i. How pleased are you with the way your abilities are used?
j. How pleased are you with the physical working conditions?
k. How pleased are you with your job as a whole, everything taken into consideration?
l. How pleased are you with your salary?
m. Does your manager is good at work planning?
n. Does your manager give high priority to job satisfaction?
o. Does your manager is good at solving conflicts?
p. Does your manager ensure that staff have good development opportunities?
q. Is there a good atmosphere between you and your colleagues?
r. Do the employees withhold information from each other?
s. Do the employees in general trust each other?
t. Do the employees withhold information from the management?
u. Does the management withhold important information from the employees?
v. Can the employees trust the information that comes from the management?
w. Does the management trust the employees to do their work well?
x. Are the employees able to express their views and feelings?
y. At my work, I feel bursting with energy.
z. I am immersed in my work.
aa. I am enthusiastic about my job.

### Training

Not all features extracted during the feature engineering step are relevant to every question. For example, the number of internal events an individual has may be more correlated with questions about Horizontal Trust within the workplace, whereas the number of external emails they send outside working hours may have more of a correlation with Burnout questions. Thus, embodiments of the system and method implement functionality in Python using Scikit-Learn which analyzes the features and their relevance to specific questions, in order to remove irrelevant features and keep only the most useful ones.

Through a process of exploratory data analysis and using the feature selection functionality discussed above, real-life feature and label data (retrieved from S3) is used as input for a variety of ML algorithms, including but not limited to Logistic Regression, XGBoost, Random Forest, and Neural Networks. These algorithms are trained to learn what communication features correspond to what survey answers, and how to map the input to the output. Performance of the trained models is assessed using a variety of metrics such as accuracy, recall, precision, and F1-score. The best-performing models for each question are then saved as Python objects to S3 to be used for inference later. This model training and testing step is performed on AWS SageMaker using Python and its data science libraries like Pandas, Scikit-Learn, and TensorFlow.

Model training and validation is an iterative process, and further steps in feature engineering and model design, as well as more labelled data collected from Pulse, will result in increased performance and more robust models.

### Prediction

Trained models with suitable performance are retrieved from S3 and deployed using SageMaker. These model objects are set up for inference using an https endpoint for real-time predictions. Nightly, this endpoint requests input data from S3, passes it through each question's trained model (which make the predictions), and stores each prediction as a separate record in a survey answer table. These predicted records mimic the format of actual survey results for the sake of the unified data model. Also note that the input data for this step consists of processed communication data for each employee of an organization, solely for those whom embodiments of the methods and systems do not already have real survey data from Pulse.

Predicted answers are saved by appending them to existing survey answers table on Redshift (with an identifier indicating the source of said answers). They are then used for the sake of displaying results to customers, through dashboards on the Hölmetrics^{™} Web App - more information below.

### Data Pipelines and Data Warehouse

A data warehouse is shown in Figs. 4A and 4B which serves data required for front-end web applications and dashboards. Data in the data warehouse also serves as input into data science models and is also a location where the outputs (or survey result predictions) from data science models will be stored. The data warehouse may be hosted on an Amazon^{™} Redshift cluster and provides a dimensional model that is easy to report off of and provides for easy transfer to the front-end data layer hosted, for example, in Good Data workspace (https://www.gooddata.com/).

### Good Data LDM

The final resting zone for data resides in business intelligence tool (Good Data). A final layer of processing occurs on the data from the data warehouse to segregate the data and store it nightly in individual workspaces (separated by organization) in the Good Data logical data model. The logical data model provides for easy presentment on the dashboards using Good Data's embedded dashboard capabilities.

### Hölmetrics^{™} Pulse

To train and validate the model, labelled data is used, which is responses by employees to survey questions derived from COPSOQ (Copenhagen Psychosocial Questionnaire). Therefore, COPSOQ serves as the basis for the labelled data, and it also is the output data of data science modelling. Meaning, the models are in fact predicting how employees would answer the COPSOQ daily based on the data points that are extracted from their internal and external communication. Although embodiments of the system and method use COPSOQ, any similar framework could be used to train a validate AI/ML to predict how employees would answer survey questions.

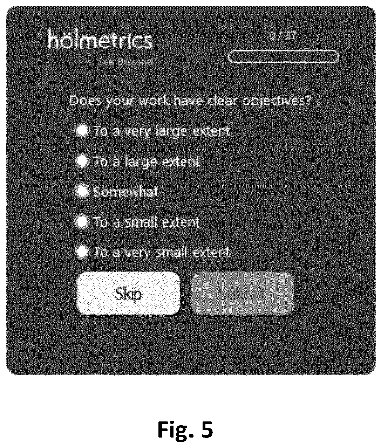

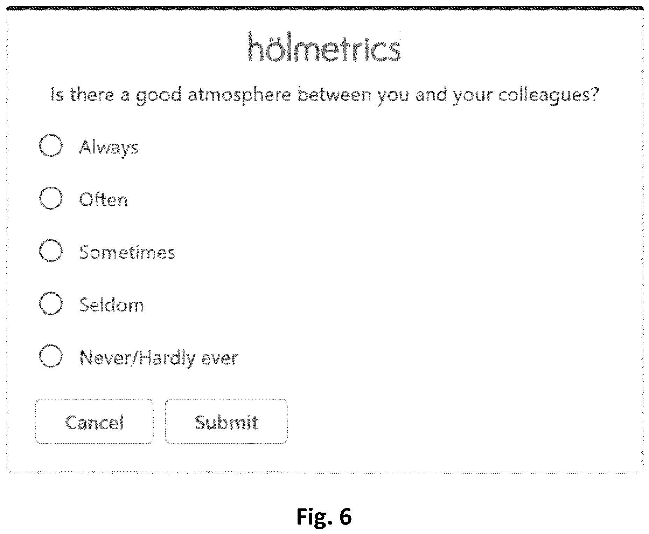

Hölmetrics^{™} Pulse provides the mechanism by which the survey data from employees is collected. There are four different exemplary methods provided by which to collect the Pulse data depending on customer preferences.
1) Intranet widget: An iframe can be used to host a widget-like front end to employees, which will ask them a configurable number of questions per day, such as set out in the example in Fig. 5. Typically, this would reside on a corporate intranet site or some other site which is regularly frequented by employees. The responses submitted by employees are sent to the Widget API service which collects and stores the data in the survey_results table.
2) Microsoft Teams^{™} Adaptive Cards: A python script (wrapped in an AWS lambda function) coupled with an Azure Bot and Bot Service sends adaptive card Pulse surveys to employees in a configurable frequency for example as shown in Fig. 6. The responses submitted by employees are sent to the Widget API service which collects and stores the data in the survey_results table. The Teams^{™} app generated using the method described herein is currently validated and published by Microsoft^{™}.
3) Slack^{™} App: Similar to the Microsoft Teams^{™} adaptive cards. A slack Pulse survey can be sent to employees with configurable frequency to employees. The responses submitted by employees are sent to the Widget API service which collects and stores the data in the survey _results table.

### Hölmetrics^{™} Web Application

The Hölmetrics^{™} Web App serves as the customer facing front end of the entire solution. From this front end users/employees of customers can view relevant data including scoring on the Hölmetrics^{™} risk and growth factors, trending, and drill down and filter capability. The web app also provides a resource centre for relevant documentation and videos as well as methods to reach out for application support. The scoring is based on a combination of real responses from employees through Pulse AND from predicted responses from the AI/ML models.

### Login Page:

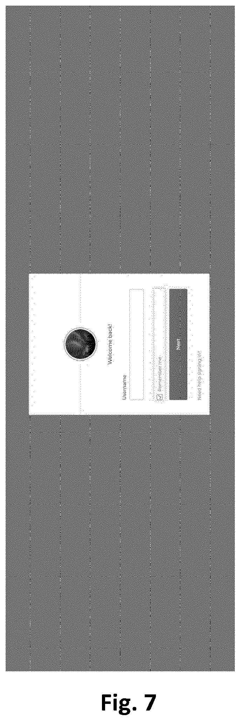

Authentication into the application can be done through SSO (provider is Okta). Users can enter their Windows^{™} credentials to enter the application as shown in Fig. 7. The application will remember their credentials the next time the login and they will be automatically authenticated.

### Overview Section

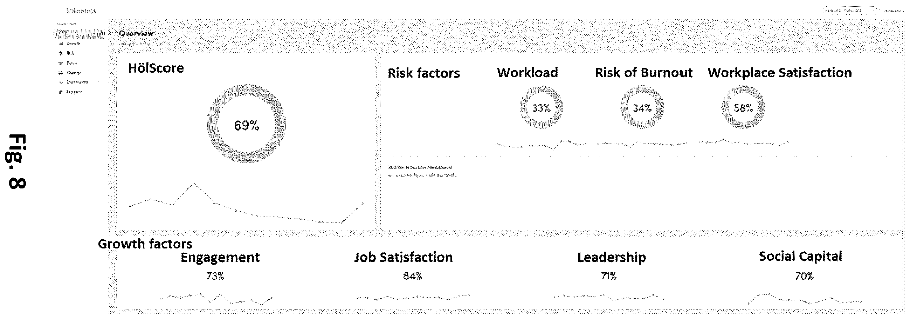

The overview section as shown in Fig. 8 provides aggregated scores for the customers. It presents the risk factors (for example, Workload, Risk of Burnout) and the Growth Factors (for example, Engagement, Job Satisfaction, Leadership, Social Capital). Trendlines allow you to see how these factors change over time.

### Growth Factors

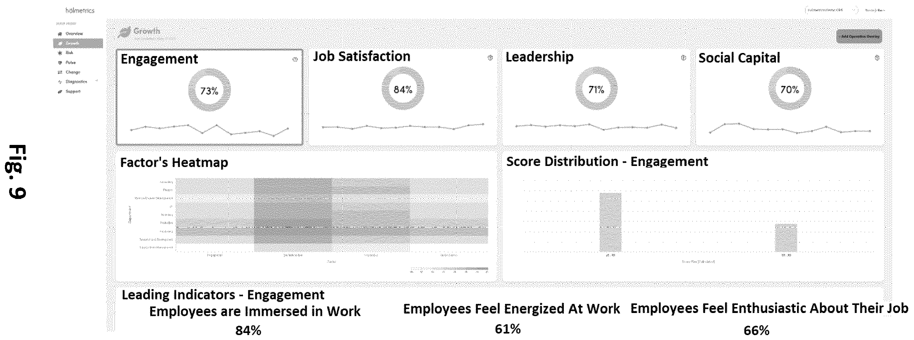

Fig. 9 shows an example of the display which provides deeper analysis into growth factors such as heatmaps by departments, score distributions, and a breakdown of leading indicators for each of the growth factors. This page also allows the user to create operational overlays which will display a chart of their operational metrics alongside any number of the Hölmetrics^{™}.

### Risk Factors

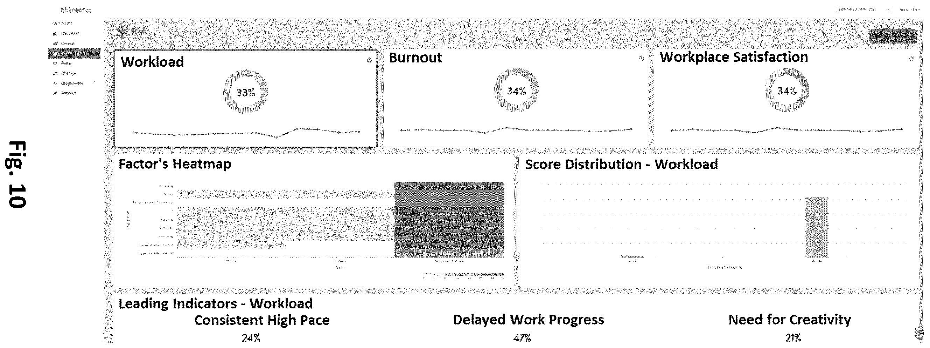

Fig. 10 shows an example of a display which provides deeper analysis into risk factors such as heatmaps by departments, score distributions, and a breakdown of leading indicators for each of the risk factors. This page also allows the user to create operational overlays which will display a chart of their operational metrics alongside any number of the Hölmetrics^{™}.

### Pulse

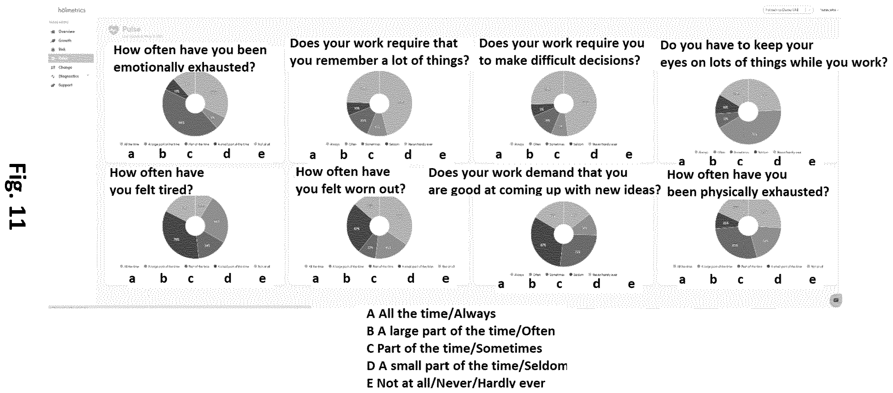

Fig. 11 shows an example of a display which provides individual results for survey questions and will provide users the ability to create action plans and comment on results publicly through MS Teams^{™} integration.

### Change

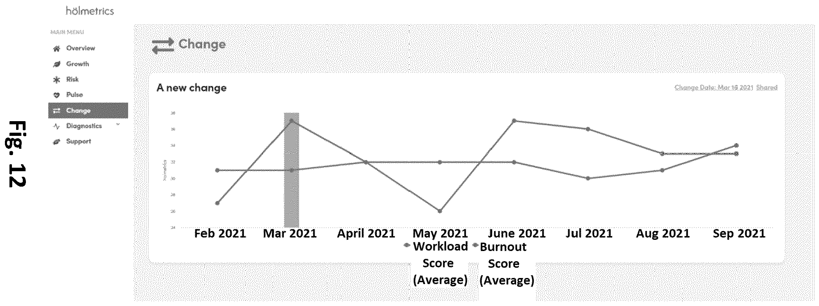

Fig. 12 shows an example of a display that allows users to select specific time periods and create charts to see how Hölmetrics^{™} factors have improved or declined during that time period (i.e. Moved office, new CEO, etc.).

### Diagnostics

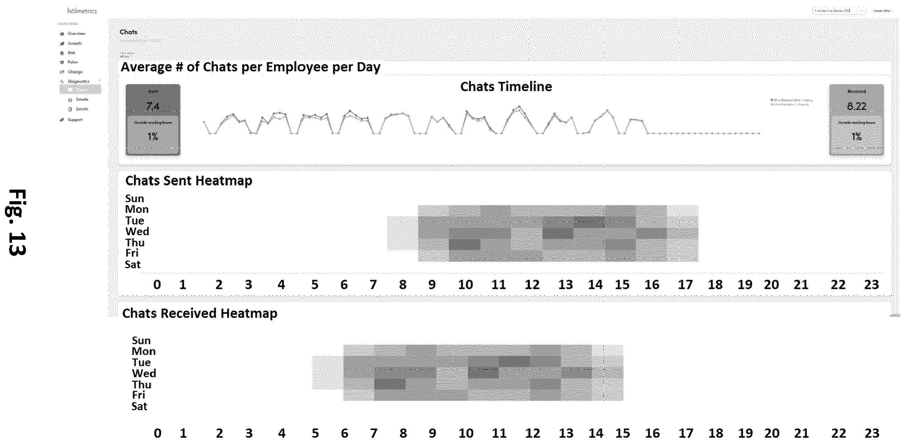

Fig. 13 shows an example of a display that allows the user to see trending and communication patterns related to Emails, Chat, and Calendar events.

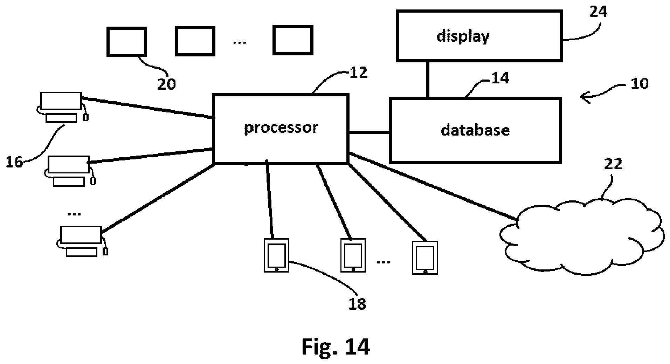

In Fig. 14, there is shown an embodiment of a system 10 for predicting employee wellness. The system 10 includes a processor 12 configured to collect information from one or more devices 16, 18, 20 associated with one or more employees in a work environment. The devices could be desktop computers, mobile phones, sensors such as CO₂, Bluetooth, temperature, etc. or any other device which includes information about an employee in a workplace. The processor may also be configured to access information stored in the cloud 22 or any other place that stores or tracks information related to an employee. A database 14 is operatively connected to the processor 12. The database 14 is configured to storing a model of a relationship between passively collected electronic data associated with one or more individuals and extracted values extracted from a subset of extracted information from the electronic data. The processor 12 is configured to derive the model of the relationship based on the collected survey data and the extracted values. The processor 12 is further configured to: passively collect further electronic data associated with one or more employees in a work environment from the one or more devices; extract a subset of extracted information from the further electronic data to create further extracted values; based on the model of the determined relationship between the collected survey data and the extracted values, predict responses to a hypothetical further survey directed to the one or more employees using the further extracted values to create predicted survey data; store the predicted survey data in the database 14; and predict employee wellness of the one or more employees by applying predicted survey data to a framework for predicting employee wellness based on actual survey data.

A display 24 may be operative connected to the processor and configured to display the predicted employee wellness on a dashboard. The processor may determine a relationship between the collected survey data and the extracted values by using artificial intelligence and machine learning to predict employee responses to survey data. The further electronic data associated with the one or more employees may be one or more of: a number of employees in an organization, the relationship between the employee and other employees in the organization, internal communications between employees at the organization, external communication between employees and third parties, calendar event data, and time zone information. The further electronic data associated with the one or more employees may be one or more of individual level data and organization level data. The further electronic data may be communication data. The processor may be configured to: collect survey data from the one or more employees, and predict employee wellness of the one or more employees based on collected survey data in addition to the predicted survey data. The passive collection of further electronic data may include performing natural language processing on the content of the collected information. The processor may be configured to verify the predicted survey data in part by comparing the predicted survey data with the collected survey data.

In this patent document, references are made to various terms such as employees and workplaces. These terms are used in their inclusive sense, and includes government organizations, charities, associations, and other large organizations and individuals who are members of these organizations, and any other groups of individuals that have significant interaction between individuals. The reference to employees or work environments is not intended to restrict the terms to only for-profit business.

The ability to compare results and predictions across two different organizations (for merger use cases, and other use cases) is also possible using embodiments of the methods and systems disclosed herein. Embodiments of the systems and methods also provide the ability of the user in the front end to create their own custom visualization based on a point in time and see results after that point in time.

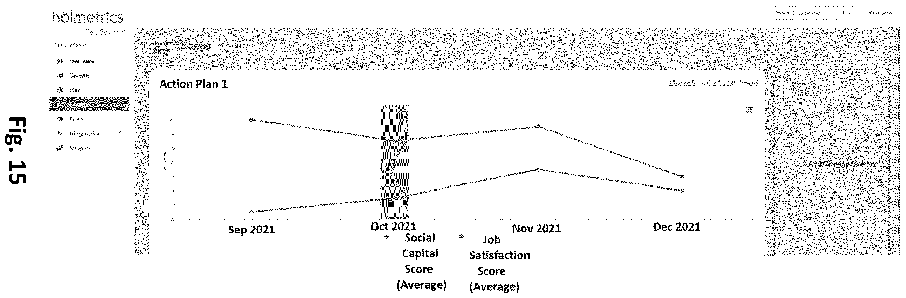

As shown in Fig. 15, the dashboard may compare results across different companies, different demographics from a point in time that is meaningful for the organization (merger date, change in management, layoffs, etc.).

Immaterial modifications may be made to the embodiments described here without departing from what is covered by the claims. For example, each of the databases or processors or other systems disclosed may be physical, virtual or cloud-based systems so long as each can implement the methods disclosed. In those cases where the systems are physical, the functionality described for each of the database or processor or other systems may be implemented by a single piece of hardware at a specific location or may make use of multiple systems at separate locations. The databases and processors themselves may be provided together on a single piece of hardware or multiple pieces of hardware. The processors may include or be associated with one or more processors that may be of any configuration so long as they are able to carry out the methods disclosed. The databases may be any storage medium that can hold accessible data as described in the methods disclosed. The network systems described may be entirely digital or analog or a mixture of the two so long as the necessary connections may be made between devices. The processors may be any computing device, or virtual machine, or combinations thereof, that allows for the ability to store and analyze the data required to implement one or more of the methods described herein.

In the claims, the word "comprising" is used in its inclusive sense and does not exclude other elements being present. The indefinite articles "a" and "an" before a claim feature do not exclude more than one of the feature being present. Each one of the individual features described here may be used in one or more embodiments and is not, by virtue only of being described here, to be construed as essential to all embodiments as defined by the claims.

## Claims

1. A method of predicting employee wellness, the method comprising:
collecting survey data from one or more individuals;
passively collecting electronic data associated with the one or more individuals;
extracting a subset of extracted information from the electronic data to create extracted values;
determining a relationship between the collected survey data and the extracted values and storing a model of the relationship in a database;
passively collecting further electronic data associated with one or more employees in a work environment;
extracting a subset of extracted information from the further electronic data to create further extracted values;
based on the model of the determined relationship between the collected survey data and the extracted values, predicting responses to a hypothetical further survey directed to the one or more employees using the further extracted values to create predicted survey data; and
predicting employee wellness of the one or more employees by applying predicted survey data to a framework for predicting employee wellness based on actual survey data.

2. The method of claim 1 further comprising displaying the predicted employee wellness to a user on a dashboard.

3. The method of any one of claims 1 or 2 further comprising implementing a change to a workplace environment based on the predicted employee wellness of the one or more employees.

4. The method of any one of claims 1 to 3 in which determining a relationship between the collected survey data and the extracted values further comprises using artificial intelligence and machine learning to predict employee responses to survey data.

5. The method of any one of claims 1 to 4 in which the further electronic data associated with the one or more employees further comprises one or more of: a number of employees in an organization, the relationship between the employee and other employees in the organization, internal communications between employees at the organization, external communication between employees and third parties, calendar event data, and time zone information.

6. The method of claim 5 in which the further electronic data associated with the one or more employees comprises individual level data and organization level data.

7. The method of any one of claims 5 or 6 in which the further electronic data further comprises communication data.

8. The method of any one of claims 1 to 7 further comprising:
collecting survey data from the one or more employees, and
predicting employee wellness of the one or more employees based on collected survey data in addition to the predicted survey data.

9. The method of any one of claims 1 to 8 further comprising providing one or more recommended action items based on predicted employee wellness of the one or more employees.

10. The method of claim 7 in which the passive collection of further electronic data further comprises performing natural language processing on the content of the collected information.

11. The method of claim 8 further comprising verifying the predicted survey data in part by comparing the predicted survey data with the collected survey data.

12. A system for predicting employee wellness, the system comprising:
a processor configured to collect information from one or more devices associated with one or more employees in a work environment;
a database operatively connected to the processor, the database configured to storing a model of a relationship between passively collected electronic data associated with one or more individuals and extracted values extracted from a subset of extracted information from the electronic data;
the processor configured to derive the model of the relationship based on the collected survey data and the extracted values;
the processor further configured to:
passively collect further electronic data associated with one or more employees in a work environment from the one or more devices;
extract a subset of extracted information from the further electronic data to create further extracted values;
based on the model of the determined relationship between the collected survey data and the extracted values, predict responses to a hypothetical further survey directed to the one or more employees using the further extracted values to create predicted survey data;
store the predicted survey data in the database; and
predict employee wellness of the one or more employees by applying predicted survey data to a framework for predicting employee wellness based on actual survey data.

13. The system of claim 12 further comprising a display operative connected to the processor and configured to displaying the predicted employee wellness on a dashboard.

14. The system of claim 12 or 13 in which the processor determining a relationship between the collected survey data and the extracted values further comprises using artificial intelligence and machine learning to predict employee responses to survey data.

15. The system of any one of claims 12 to 14 in which the further electronic data associated with the one or more employees further comprises one or more of: a number of employees in an organization, the relationship between the employee and other employees in the organization, internal communications between employees at the organization, external communication between employees and third parties, calendar event data, and time zone information.

16. The system of claim 15 in which the further electronic data associated with the one or more employees comprises individual level data and organization level data.

17. The system of any one of claims 15 or 16 in which the further electronic data further comprises communication data.

18. The system of any one of claims 12 to 17 in which the processor is further configured to:
collect survey data from the one or more employees, and
predict employee wellness of the one or more employees based on collected survey data in addition to the predicted survey data.

19. The system of any one of claims 12 to 18 in which the passive collection of further electronic data further comprises performing natural language processing on the content of the collected information.

20. The system of claim 18 further comprising the processor being configured to verify the predicted survey data in part by comparing the predicted survey data with the collected survey data.
